# EUROPÄISCHE PATENTSCHRIFT

(11) **EP 0 478 837 B1**
(45) Veröffentlichungstag und Bekanntmachung des Hinweises auf die Patenterteilung: **25.01.1995**
(21) Anmeldenummer: 90119074.4
(22) Anmeldetag: 04.10.1990
(51) Int. Cl.: C13K 13/00, A23L 1/0526, A23L 1/308, A61K 31/70

(54) **Lactulosezubereitung und Verfahren zu ihrer Herstellung**
Lactulose preparation and its manufacturing process
Préparation de lactulose et son procédé de fabrication

(43) Veröffentlichungstag der Anmeldung: 08.04.1992
(73) Patentinhaber: Laevosan-Gesellschaft m.b.H., A-4020 Linz (AT)
(72) Erfinder: Nitsch, Ernst, A-4040 Linz (AT)
(74) Vertreter: Huber, Bernhard, Dipl.-Chem.

(56) Entgegenhaltungen:
- EP-A- 0 189 722
- FR-M- 212
- US-A- 2 060 336

## Beschreibung

Die Erfindung betrifft eine Lactulosezubereitung sowie ein Verfahren zu ihrer Herstellung.

Lactulose ist ein nicht natürlich vorkommendes Disaccharid mit der Struktur
4-β-D-Galactosido-D-fructose.

Lactulose läßt sich nach bekannten Verfahren leicht aus Lactose durch Isomerisierung herstellen. Man erhält dann Lösungen, die überwiegend Lactulose neben Lactose und anderen Nebenprodukten enthalten. Die Schwierigkeit besteht nun darin, die Lactulose in eine Form zu bringen, die sich für die therapeutische Anwendung eignet. Schwierigkeiten bereitet die Herstellung von Lactulose in fester Form. Hierzu sind sehr aufwendige Verfahren erforderlich, da die Lactulose aus unreinen Lösungen sehr schwer kristallisiert und andererseits die durch Totaltrocknung erhaltenen Pulver hochhygroskopisch sind und daher schnell verklumpen. Hochkonzentrierte Lactuloselösungen sind hochviskos und daher für eine Verabreichung nicht geeignet. Es war daher bisher üblich, Lactulose in Form von ca. 50% Lactulose enthaltenden Lösungen als Sirup mit ca. 65% Gesamtzuckergehalt zu verwenden. Nachteil dabei ist es, daß man zum Erreichen der therapeutischen Dosis sehr hohe Mengen dieses Sirups verabreichen muß, was wegen des sehr süßen aufdringlichen Geschmackes unangenehm ist. Es wurde nun auch schon versucht, durch Zusatz von Hilfsstoffen brauchbare Trockenprodukte direkt aus dem Sirup herzustellen. So wurde beispielsweise in US-PS 3,110,600 vorgeschlagen, eine Sprühtrocknung unter Zusatz von 2 bis 5 % eines verkleisterten Getreidemehles durchzuführen. Weiterhin wurde in DE-OS 21 53 106 vorgeschlagen, Lactulose-Trockenprodukte herzustellen durch Zumischen von mindestens 5 % Protein, vornehmlich Casein. Zum selben Zweck wird in DE-AS 21 48 159 das Zumischen von 0,3 % Konnyakupulver empfohlen. Aufgrund der erforderlichen Sprüh- oder Vakuumtrocknung ist aber immer noch ein relativ hoher Verfahrensaufwand nötig.

Eine andere Lösung wird in DE-OS 27 17 707 vorgeschlagen, worin Lactulose durch Verkochen mit Zusatzstoffen wie Fetten und Milch karamellisiert wird, wobei Bonbons, Granulate oder Pulver erhalten werden. Dieses Verfahren bedingt jedoch eine hohe thermische Belastung, die zu teilweiser Zersetzung des Wirkstoffes und zu undefinierten Veränderungen bei den Begleitstoffen führt. Dieselben Nachteile hat ein in US-PS 4,605,646 beschriebenes Verfahren, bei dem ein Lactulose-Sirup zu Bisquits mit ca. 10 % Lactulose unter Zusatz von Weizenkleie verarbeitet wird. Zusätzlicher Nachteil dieser Präparation ist der hohe kalorische Wert des benötigten Mehles und der Stärke, sowie die für eine sichere Wirkung nötige unerwünscht hohe Dosierung von mindestens 100 g pro Tag. In EP-A 0 334 407 wird weiterhin vorgeschlagen, Lactulosesirup nach Zusatz von 150 bis 350 g/l einer gereinigten Getreidekleie in dünner Schicht bei 60 bis 90°, vorzugsweise bei 80°C über 6 Stunden zu trocknen. Auch hier ist Nachteil die hohe thermische Belastung mit Gefahr der Bildung undefinierter Begleitstoffe. Ein weiteres Verfahren, das ohne Trocknung auskommt, wird in EP-A 0 189 722 beschrieben. Hier wird ein Lactulose-Gelee hergestellt durch Erhitzen von Lactulose-Sirup mit Pectin, einer Säure und Konservierungsmittel. Bei dem niedrigen anzuwendenden pH wird die Lactulose teilweise hydrolysiert. Weiterhin ergeben sich unerwünschte Geschmacks- und Farbveränderungen.

Es war nun Aufgabe der vorliegenden Erfindung, ein Lactulosepräparat zur Verfügung zu stellen, das für die therapeutische Anwendung geeignet ist, in einfacher Weise ohne thermische Belastung und ohne apparativen Aufwand hergestellt werden kann und einfach zu dosieren ist.

Diese Aufgabe wird gelöst durch eine Lactulosezubereitung, die dadurch gekennzeichnet ist, daß sie in Gelform vorliegt und Lactulose und eine pflanzliche Schleimdroge sowie gegebenenfalls übliche pharmazeutische Zusatzstoffe oder/und Antiflatulenzmittel enthält.

Überraschenderweise gelingt es, Lactuloselösung zum Erstarren zu bringen durch den Zusatz einer Schleimdroge. Es werden dabei geleeartige Zubereitungen erhalten, die angenehm einzunehmen sind, sich leicht dosieren lassen und ohne großen Aufwand hergestellt werden können.

Die erfindungsgemäße Zubereitung enthält Lactulose und eine oder mehrere Schleimdrogen. Als Schleimdroge wird geeigneterweise eine solche verwendet, die in bezug auf die laxierende Wirkung synergistisch wirkt. Bevorzugte Beispiele hierfür sind Samen oder/und Samenschalen von Plantago ovata L., Plantago Psyllium L., Plantago indica L., auch als Flohsamen bezeichnet, und/oder Linum usitatissimum L., auch als Leinsamen bezeichnet, die allein oder in Mischung verwendet werden.

Zur Herstellung der Zubereitung wird die Lactulose in Form der Lösung eingesetzt, wobei bevorzugt die handelsüblichen Lactuloselösungen mit einem Lactulose-Gehalt im Bereich von 40 bis 70 % verwendet werden. Besonders bevorzugt wird die Lactulose in Form einer ca. 50% Lactulose neben gegebenenfalls weiteren Zuckern enthaltenden Lösung eingesetzt.

Die erfindungsgemäße Lactulosezubereitung kann noch weitere Zusatzstoffe enthalten. Zum einen können, abhängig von der gewünschten Anwendung, dem Präparat übliche Träger- und Verdünnungsmittel zugesetzt werden, um es in die gewünschte Form zu bringen. Andererseits können für den gewünschten Anwendungszweck zusätzliche Wirkstoffe eingesetzt werden. Eine bei Anwendung von Lactulose häufige Nebenwirkung ist Flatulenz, so daß geeigneterweise das erfindungsgemäße Mittel zusätzlich noch ein gegen Flatulenz wirksames Mittel wie z.B. ein hierzu wirksames Silikonöl - Dimethicone - enthält.

Bevorzugt wird die erfindungsgemäße Lactulosezubereitung zur Behandlung der bekanntermaßen mit Lactulose behandelten Gesundheitsstörungen oral verabreicht.

Durch den hohen Zuckergehalt und die dadurch geringe Wasseraktivität können Mikroorganismen in Gegenwart der erfindungsgemäßen Zubereitung nicht wachsen. Die Zubereitung muß daher nicht konserviert werden.

Bevorzugt erfolgt die Herstellung so, daß man einer Lactuloselösung ein Pulver einer Schleimdroge zusetzt, beide Komponenten mischt und dann erstarren läßt.

Auf diese Weise läßt sich die Zubereitung ohne Anwendung von Wärme, ohne Trocknungsschritt und ohne pH-Änderung leicht herstellen. Weder eine Verdünnung noch eine Konzentrierung des Sirups ist erforderlich. Durch einfaches Ausgießen der noch flüssigen Mischung in beliebige Formen können Zubereitungsformen der gewünschten Art und in der gewünschten Dosierung problemlos zubereitet werden. Es lassen sich ebenso problemlos weitere Wirkstoffe einarbeiten, auch solche, die in Wasser unlöslich sind, indem einfach der Wirkstoff einer der beiden Komponenten oder der Mischung vor dem Erstarrenlassen zugesetzt wird.

Die Erfindung wird durch die folgenden Beispiele erläutert.

### Beispiel 1

### Lactulosegelee mit Flohsamen

In den Mixbecher einer Küchenmaschine wurden 420 g Lactulose-Konzentrat mit einem Gehalt von 50 % Lactulose (Qualität entspricht British Pharmacopoeia 88) vorgelegt, 90 g gemahlener Flohsamen und 1,4 g Pflaumenaroma zugegeben und für ca. eine Minute homogenisiert. Anschließend wurde die noch fließfähige Masse in halbkugelige Formen mit Einzeldosis von je ca. 17 g ausgegossen. Die Masse erstarrte innerhalb von 10 Minuten zu einem weichen Gel. Innerhalb von weiteren 24 Stunden wurde die endgültige Konsistenz eines schneidfähigen dicken Fruchtmuses erreicht. Jede Einzeldosis enthielt ca. 7 g Lactulose und 3 g Flohsamen. Die übliche Tagesdosis für Erwachsene besteht aus 3 Einzeldosen. Das Präparat weist einen angenehm süß-fruchtigen Geschmack auf.

### Beispiel 2

### Lactulosegelee mit Flohsamen und Antiflatulenz-Wirkstoff

Zu einer Mischung von Beispiel 1 wurden vor der Homogenisierung zusätzlich 1,2 g Dimethicone (Silikonöl, gemäß European Pharmacopoeia 2nd Edition), ein hochwirksames Antiflatulenzmittel, zugegeben. Verarbeitung, Konsistenz und Dosierung wie in Beispiel 1.

### Beispiel 3

### Lactulosegelee mit Leinsamen

In den Mixbecher einer Küchenmaschine wurden 420 g Lactulose-Konzentrat mit einem Gehalt von 50 % Lactulose vorgelegt, 135 g entfetteter gemahlener Leinsamen und 1,4 g Pflaumenaroma zugegeben und für ca. eine Minute homogenisiert. Anschließend wurde die noch fließfähige Masse in halbkugelige Formen mit Einzeldosis von je ca. 18,5 g ausgegossen. Konsistenz und Geschmack waren ähnlich wie in Beispiel 1. Jede Einzeldosis enthält ca. 7 g Lactulose und 4,5 g Leinsamen.

### Beispiel 4

### Lactulosegelee mit Leinsamen und Antiflatulenz-Wirkstoff

Zu der Mischung von Beispiel 3 wurden vor der Homogenisierung zusätzlich 1,2 g Dimethicone, ein hochwirksames Antiflatulenzmittel zugegeben. Verarbeitung, Konsistenz und Dosierung wie in Beispiel 1.

### Beispiel 5

### Lactulosegelee mit Samenschalen von Flohsamen

Wie in Beispiel 1 wurden in 420 g Lactulose-Konzentrat 90 g gepulverte Samenschalen von Flohsamen eingearbeitet.
Gewicht der Einzeldosis: ca. 17 g.
Diese enthält ca. 7 g Lactulose und 3 g Flohsamen-Samenschalen. Das Präparat ist von etwas festerer Konsistenz als in Beispiel 1 und von hellerer Farbe.

### Beispiel 6

| Mischung Nr. | Lactulose-Konzentrat 50 % Lactulose g | Flohsamen gepulvert g | Flohsamen-Samenschale gepulvert g | Leinsamen entfettet gepulvert g | Dimethicone g | Einzeldosis g |
|---|---|---|---|---|---|---|
| 1 | 420 | 45 | - | 67,5 | - | 17,75 |
| 2 | 420 | 45 | - | 67,5 | 1,2 | 17,75 |
| 3 | 420 | 60 | 30 | - | - | 17 |
| 4 | 420 | 60 | 30 | - | 1,2 | 17 |

Alle Mischungen wurden analog Beispiel 1 oder 2 hergestellt und hatten ähnliche Eigenschaften wie die dort beschriebenen Produkte.

## Patentansprüche (Patentansprüche für folgende(n) Vertragsstaat(en): AT, BE, CH, DE, DK, FR, GB, IT, LI, LU, NL, SE)

1. Lactulosezubereitung,
**dadurch gekennzeichnet**,
daß sie in Gelform vorliegt und Lactulose und eine pflanzliche Schleimdroge sowie gegebenenfalls übliche pharmazeutische Zusatzstoffe oder/und Antiflatulenzmittel enthält.

2. Lactulosezubereitung nach Anspruch 1,
**dadurch gekennzeichnet**,
daß sie 30 bis 50 Gew.-% Lactulose enthält.

3. Lactulosezubereitung nach Anspruch 1 oder 2,
**dadurch gekennzeichnet**,
daß sie als Schleimdroge Samen oder/und Samenschalen von Plantago ovata L., Plantago Psyllium L., Plantago indica L. oder von Linum usitatissimum L. enthält.

4. Lactulosezubereitung nach einem der vorhergehenden Ansprüche, **dadurch gekennzeichnet**, daß sie die Schleimdroge in einer Konzentration von 10 bis 30 % enthält.

5. Lactulosezubereitung nach einem der vorhergehenden Ansprüche, **dadurch gekennzeichnet**, daß sie in Geleeform vorliegt.

6. Verfahren zur Herstellung einer Lactulosezubereitung, **dadurch gekennzeichnet**, daß man einer Lactuloselösung ein Pulver einer Schleimdroge zusetzt, beide Komponenten mischt und dann erstarren läßt.

7. Verfahren nach Anspruch 6, **dadurch gekennzeichnet**, daß man eine Lactuloselösung mit einem Gehalt von 40 bis 70 % Lactulose verwendet.

8. Verfahren nach Anspruch 6 oder 7,
**dadurch gekennzeichnet**, daß man die Schleimdroge in Form eines gemahlenen und gegebenenfalls entfetteten Pulvers einsetzt.

9. Verfahren nach einem der Ansprüche 6 bis 8,
**dadurch gekennzeichnet**, daß man als Schleimdroge Samen oder/und Samenschalen von Plantago ovata L., Plantago Psyllium L., Plantago indica L. oder von Linum usitatissimum L. einsetzt.

10. Verfahren nach einem der Ansprüche 6 bis 9,
**dadurch gekennzeichnet**, daß man das Mischen der Komponenten bei Raumtemperatur durchführt.

11. Verfahren nach einem der Ansprüche 6 bis 10,
**dadurch gekennzeichnet**, daß man der Mischung vor dem erstarrenlassen weitere Wirkstoffe und/oder übliche pharmazeutische Träger- und Verdünnungsmittel zufügt.

12. Verfahren nach Anspruch 11, **dadurch gekennzeichnet**, daß man der Mischung als Antifaltulanzmittel Silikonöl zufügt.

## Patentansprüche (Patentansprüche für folgende(n) Vertragsstaat(en): ES, GR)

1. Verfahren zur Herstellung einer Lactulosezubereitung, **dadurch gekennzeichnet,** daß man einer Lactuloselösung ein Pulver einer Schleimdroge zusetzt, beide Komponenten mischt und dann erstarren läßt.

2. Verfahren nach Anspruch 1, **dadurch gekennzeichnet,** daß man eine Lactuloselösung mit einem Gehalt von 40 bis 70 % Lactulose verwendet.

3. Verfahren nach Anspruch 1 oder 2,
**dadurch gekennzeichnet,** daß man die Schleimdroge in Form eines gemahlenen und gegebenenfalls entfetteten Pulvers einsetzt.

4. Verfahren nach einem der Ansprüche 1 bis 3,
**dadurch gekennzeichnet,** daß man als Schleimdroge Samen oder/und Samenschalen von Plantago ovata L., Plantago Psyllium L., Plantago indica L. oder von Linum usitatissimum L. einsetzt.

5. Verfahren nach einem der Ansprüche 1 bis 4,
**dadurch gekennzeichnet,** daß man das Mischen der Komponenten bei Raumtemperatur durchführt.

6. Verfahren nach einem der Ansprüche 1 bis 5, **dadurch gekennzeichnet,** daß man der Mischung vor dem erstarrenlassen weitere Wirkstoffe und/oder übliche pharmazeutische Träger- und Verdünnungsmittel zufügt.

7. Verfahren nach Anspruch 6, **dadurch gekennzeichnet,** daß man der Mischung als Antifaltulanzmittel Silikonöl zufügt.

## Claims (Claims for the following Contracting State(s): AT, BE, CH, DE, DK, FR, GB, IT, LI, LU, NL, SE)

1. A lactulose preparation, characterised in that it is present in the form of a gel and contains lactulose and a vegetable mucilage drug, as well as, optionally, conventional pharmaceutical additives and/or antiflatulence agents.

2. A lactulose preparation according to Claim 1, characterised in that it contains 30 to 50 % by weight of lactulose.

3. A lactulose preparation according to Claim 1 or 2, characterised in that as mucilage drug it contains seeds and/or seed husks of Plantago ovata L., Plantago Psyllium L., Plantago indica L. or of Linum usitatissimum L.

4. A lactulose preparation according to any one of the preceding Claims, characterised in that it contains the mucilage drug in a concentration of from 10 to 30 %.

5. A lactulose preparation according to any one of the preceding Claims, characterised in that it is present in the form of a jelly.

6. A method of preparing a lactulose preparation, characterised in that a powder of a mucilage drug is added to a lactulose solution, both components are mixed and then allowed to solidify.

7. A method according to Claim 6, characterised in that a lactulose preparation containing from 40 to 70 % lactulose is used.

8. A method according to Claim 6 or 7, characterised in that the mucilage drug is used in the form of a ground and optionally degreased powder.

9. A method according to any one of Claims 6 to 8, characterised in that seeds and/or seed husks of Plantago ovata L., Plantago Pzyllium L., Plantago indica L. or of Linum uzitatissimum L. are used as mucilage drug.

10. A method according to any one of Claims 6 to 9, characterised in that the mixing of the components is carried out at room temperature.

11. A method according to any one of Claims 6 to 10, characterised in that, before being allowed to solidify, further active substances and/or conventional pharmaceutical excipients and diluents are added to the mixture.

12. A method according to Claim 11, characterised in that silicone oil is added to the mixture as antiflatulence agent.

## Claims (Claims for the following Contracting State(s): ES, GR)

1. A method of preparing a lactulose preparation, characterised in that a powder of a mucilage drug is added to a lactulose solution, both components are mixed and then allowed to solidify.

2. A method according to Claim 1, characterised in that a lactulose solution is used containing from 40 to 70 % lactulose.

3. A method according to Claim 1 or 2, characterised in that the mucilage drug is used in the form of a ground and optionally degreased powder.

4. A method according to any one of Claims 1 to 3, characterised in that seeds and/or seed husks of Plantago ovata L., Plantago Psyllium L., Plantago indica L. or of Linum usitatissimum L. are used as mucilage drug.

5. A method according to any one of Claims 1 to 4, characterised in that the mixing of the components is carried out at room temperature.

6. A method according to any one of Claims 1 to 5, characterised in that, before being allowed to solidify, further active substances and/or conventional pharmaceutical excipients and diluents are added to the mixture.

7. A method according to Claim 6, characterised in that silicone oil is added to the mixture as antiflatulence agent.

## Revendications (Revendications pour l'(les) Etat(s) contractant(s) suivant(s): AT, BE, CH, DE, DK, FR, GB, IT, LI, LU, NL, SE)

1. Préparation de lactulose, caractérisée en ce qu'elle se présente sous forme de gel et contient du lactulose et une drogue mucilagineuse végétale ainsi qu'éventuellement des adjuvants pharmaceutiques usuels et/ou des antiflatulents.

2. Préparation de lactulose selon la revendication 1, caractérisée en ce qu'elle contient 30 à 50% en poids de lactulose.

3. Préparation de lactulose selon la revendication 1 ou 2, caractérisée en ce qu'elle contient en tant que drogue mucilagineuse des graines ou/et des enveloppes de graines de Plantago ovata L., de Plantago Psyllium L., de Plantago indica L. ou de Linum usitatissimum L.

4. Préparation de lactulose selon l'une des revendications précédentes, caractérisée en ce qu'elle contient la drogue mucilagineuse à une concentration de 10 à 30%.

5. Préparation de lactulose selon l'une des revendications précédentes, caractérisée en ce qu'elle se présente sous forme de gelée.

6. Procédé de préparation d'une composition de lactulose, caractérisé en ce qu'on ajoute à une solution de lactulose une poudre d'une drogue mucilagineuse, mélange les deux composants et laisse ensuite prendre en masse.

7. Procédé selon la revendication 6, caractérisé en ce qu'on emploie une solution de lactulose avec une teneur en lactulose de 40 à 70%.

8. Procédé selon la revendication 6 ou 7, caractérisé en ce qu'on met en oeuvre la drogue mucilagineuse sous forme d'une poudre broyée et, au besoin dégraissée.

9. Procédé selon l'une des revendications 6 à 8, caractérisé en ce qu'on met en oeuvre en tant que drogue mucilagineuse des graines ou/et des enveloppes de graines de Plantago ovata L., de Plantago Psyllium L., de Plantago indica L. ou de Linum usitatissimum L.

10. Procédé selon l'une des revendications 6 à 9, caractérisé en ce qu'on effectue le mélange des composants à la température ambiante.

11. Procédé selon l'une des revendications 6 à 10, caractérisé en ce qu'on ajoute au mélange, avant la prise en masse, des principes actifs additionnels et/ou les excipients et diluants pharmaceutiques usuels.

12. Procédé selon la revendication 11, caractérisé en ce qu'on ajoute au mélange une huile de silicone en tant qu'antiflatulent.

## Revendications (Revendications pour l'(les) Etat(s) contractant(s) suivant(s): ES, GR)

1. Procédé de préparation d'une composition de lactulose, caractérisé en ce qu'on ajoute à une solution de lactulose une poudre d'une drogue mucilagineuse, mélange les deux composants et laisse ensuite prendre en masse.

2. Procédé selon la revendication 1, caractérisé en ce qu'on emploie une solution de lactulose avec une teneur en lactulose de 40 à 70%.

3. Procédé selon la revendication 1 ou 2, caractérisé en ce qu'on met en oeuvre la drogue mucilagineuse sous forme d'une poudre broyée et, au besoin dégraissée.

4. Procédé selon l'une des revendications 1 à 3, caractérisé en ce qu'on met en oeuvre en tant que drogue mucilagineuse des graines ou/et des enveloppes de graines de Plantago ovata L., de Plantago Psyllium L., de Plantago indica L. ou de Linum usitatissimum L.

5. Procédé selon l'une des revendications 1 à 4, caractérisé en ce qu'on effectue le mélange des composants à la température ambiante.

6. Procédé selon l'une des revendications 1 à 5 , caractérisé en ce qu'on ajoute au mélange, avant la prise en masse, des principes actifs additionnels et/ou les excipients et diluants pharmaceutiques usuels.

7. Procédé selon la revendication 6, caractérisé en ce qu'on ajoute au mélange une huile de silicone en tant qu'antiflatulent.
